# EUROPÄISCHE PATENTSCHRIFT

(11) **EP 1 326 624 B1**
(45) Veröffentlichungstag und Bekanntmachung des Hinweises auf die Patenterteilung: **08.09.2004**
(21) Anmeldenummer: 01982401.0
(22) Anmeldetag: 05.10.2001
(51) Int. Cl.: A61K 35/78

(54) **VERFAHREN ZUR HERSTELLUNG VON PHYLLANTHUSEXTRAKTEN**
METHOD FOR PRODUCING PHYLLANTHUS EXTRACTS
PROCEDE DE PRODUCTION D'EXTRAITS DE PHYLLANTHUS

(30) Priorität: 06.10.2000 EP 00121852; 15.01.2001 EP 00100825
(43) Veröffentlichungstag der Anmeldung: 16.07.2003
(73) Patentinhaber: Phytrix AG, 80335 München (DE)
(72) Erfinder: KREUTER, Matthias-Heinrich, CH-8880 Walenstadt (CH); WAGNER, Hildebert, K., M., 83254 Breitbrunn a. Chiemsee (DE); TITTEL, Gerolf, 82166 Gräfelfing (DE)
(74) Vertreter: VOSSIUS & PARTNER
(86) Internationale Anmeldenummer: PCT/EP2001/011526
(87) Internationale Veröffentlichungsnummer: WO 2002/030436

(56) Entgegenhaltungen:
- DATABASE BIOSIS [Online] BIOSCIENCES INFORMATION SERVICE, PHILADELPHIA, PA, US; 1993 MUNSHI ANUPAMA ET AL: "Evaluation of anti-hepadnavirus activity of Phyllanthus amarus and Phyllanthus maderaspatensis in duck hepatitis B virus carrier Pekin ducks." Database accession no. PREV199497070792 XP002189216 & JOURNAL OF MEDICAL VIROLOGY, Bd. 41, Nr. 4, 1993, Seiten 275-281, ISSN: 0146-6615
- DATABASE BIOSIS [Online] BIOSCIENCES INFORMATION SERVICE, PHILADELPHIA, PA, US; 1991 MEHROTRA R ET AL: "IN-VITRO EFFECT OF PHYLLANTHUS-AMARUS ON HEPATITIS B VIRUS" Database accession no. PREV199191116067 XP002189217 & INDIAN JOURNAL OF MEDICAL RESEARCH SECTION A, Bd. 93, Nr. MAR., 1991, Seiten 71-73, ISSN: 0970-955X

## Beschreibung

Die Erfindung betrifft ein Verfahren zur Extraktion von Phyllanthus, wobei man (a) Phyllanthusbestandteile mit einem Ethanol/Wassergemisch von 5-85 % m/m extrahiert, dem ein Schwermetallchelator in einer Konzentration von 0,001-3% m/m zugesetzt ist; (b) den in Schritt (a) erhaltenen Primärextrakt mit (ba) indischem Sterculiagummi in einer Endkonzentration von 0,5-5,0% mm, bezogen auf die Summe der Extraktivstoffe, oder (bb) einem oder mehreren Polymeren sowie impendierbaren und/oder löslichen Substanz(en) versetzt und konzentriert; und (c) den in Schritt (b) erhaltenen konzentrierten Extrakt trocknet. Das erfindungsgemäße Verfahren führt zu einer besonders hohen Ausbeute an pharmakologisch wirksamen Pflanzeninhaltsstoffen und ist deshalb für therapeutische Anwendungen von besonderem Interesse. In einer bevorzugten Ausführungsform schließt das erfindungsgemäße Verfahren einen Filtrationsschritt des Primärextraktes ein. Ferner bevorzugt ist, daß während der Extraktionsverfahrens ein Lipoid zugesetzt wird. Vorzugsweise ist der Phyllanthus Phyllanthus amarus. Die Erfindung betrifft darüber hinaus Arzneimittel, die mit dem erfindungsgemäßen Verfahren gewonnene Extrakte enthalten.

Die Pflanzengattung Phyllanthus gehört zur Unterfamilie der Phyllanthoideae, die ihrerseits zur Familie der Euphorbiaceae gerechnet wird. Die Gattung Phyllanthus umfaßt insgesamt ca. 700 bekannte Arten, die in tropischen und subtropischen Gebieten in Australien, China, den Philippinen, Thailand, Indonesien, Burma, Indien, Ost- und Westafrika sowie Nordamerika, Mexiko, Kuba, der Karibik und Venezuela beheimatet sind. Nur selten werden Vertreter dieser Gattung in den nördlichen gemäßigten Zonen angetroffen.

Pflanzen der Gattung Phyllanthus enthalten sekundäre Pflanzeninhaltsstoffe, die unter Einfluss von Licht, Temperatur, Sauerstoff und Schwermetallen oxydativen Abbauprozessen unterliegen. In dieser Hinsicht sind hydrolysierbare Gerbstoffe anzuführen, wie das Didehydrohexahydroxydiphenol Amariin oder Geraniin, ein mengenmässig dominierendes Ellagitannin. Spezifisch für die Spezies Phyllanthus amarus Schumach et Thonn ist eine zusätzliche Inhaltsstoffgruppe (Lignane), die Phyllanthine, von denen in erster Linie Phyllanthin und Hypophyllanthin zu nennen sind; ersteres dominiert mengenmässig. Den Phyllanthinen und Ellagitanninen gemeinsam ist ihr antioxydatives Reaktionsvermögen, das sie einerseits als wichtige Wirkstoffe, andererseits als leicht zerstörbar charakterisiert. Während die Ellagitannine physiko-chemisch polare und sehr gut wasserlösliche Stoffe darstellen, lösen sich die Phyllanthine bevorzugt in organischen Lösungsmitteln oder Mischungen letzterer mit Wasser. Beide Stoffklassen sind nebst einer Reihe ubiquitärer primärer und sekundärer Pflanzeninhaltsstoffe in wässrigen Zubereitungen (Infuse/Decocte), wie sie in der traditionellen Volksmedizin üblich sind, anzutreffen. Dies mag dem Nichtfachmann im Falle der Phyllanthine verwunderlich erscheinen; für den Fachmann ist dieser Befund jedoch ohne weiteres erklärbar, da Pflanzen miscellanöse, lösungsvermittelnde Stoffgemische enthalten. Komplexe Vielstoffgemische verhalten sich in Bezug auf das Löslichkeitsverhalten einzelner Komponenten daher unter Umständen völlig anders, als das für einen Inhaltsstoff allein zu erwarten wäre. Sowohl für die Droge Phyllanthus amarus selbst als auch für wässrige und alkoholische Zubereitungen (Methanol, Ethanol, Butanol) liegen eine Reihe sehr widersprüchlicher pharmakologischer Daten in vitro und in vivo vor. Insbesondere Untersuchungen in Bezug auf eine antivirale Aktivität reichen in ihrem Ergebnisspektrum von stark wirksam bis wirkungslos. Für eine umfassende Übersicht siehe Hager's Handbuch der pharmazeutischen Praxis, 5. Auflage, Folgeband 3, Drogen L-Z, Springer Verlag, 342-343.

Dieser Umstand ist nur auf den ersten Blick irritierend; eine genauere Analyse der Zubereitungen, die in den verschiedenen Untersuchungen zur Anwendung gelangten, führt sehr schnell zu dem Ergebnis, dass die gewählten Zubereitungen sich in essentiellen stofflichen Merkmalen massiv unterschieden haben.

Angesichts dieser Datenlage ist eine Vergleichbarkeit der Wirkstoffgemische nicht gegeben, vgl. M.H. Kreuter, Phytopharmaceutical Technology: Progress in Process Evaluation and Process Optimizing, Plenary Lecture, 46 th Annual Congress of the Society for Medicinal Plant Research, Wien, 1998. Basierend auf den Kenntnissen zur therapeutischen Wirksamkeit durch den "Evidence Based Use" verschiedener ethnischer Gruppen ist eine Überprüfung der "Efficacy and Safety" von Phyllanthus mittels einer klinisch vernünftig handhabbaren Darreichungsform erwünscht.

Aus dem vorab Dargestellten ergeben sich für ein solches Therapeutikum allerdings eine Reihe von Bedingungen, die zu erfüllen sind. Aus pharmakodynamischer und pharmakokinetischer Sicht ist auf eine hohe stoffliche Authentizität in Bezug auf die Stofflichkeit der archetypischen Darreichungsformen zu achten. Der Prozessweg zur Darstellung des Wirkstoffs darf nicht zur Bildung prozesstypischer Folgeprodukte führen, was zwangsweise eine vom Wirkstoffvorbild abweichende Stofflichkeit implizieren würde.

So ist bekannt, daß hydrolysierbare Gerbstoffe mit Schwermetallen unter Bildung schwerlöslicher Komplexe reagieren, hydrolyrieren, oxydieren und unlösliche makromolekulare Präzipitate bilden. Für eine Übersicht siehe Schneider G., Pharmazeutische Biologie, 2., neu bearb. u. erw. Aufl., Mannheim, Wien, Zürich, Bibliographisches Institut, 1985, 307-308. Abhängig von den Reaktionsbedingungen (Temperatur, Zeit, Sauerstoff) führt dies während des Extraktionsprozesses zu einem mehr oder weniger stark ausgeprägten Verlust nativer Ellagitannine. Im Gegensatz zur traditionellen frischen Zubereitung kleinster Aufgussmengen (Tee), die innert kürzestem Zeitraum eingenommen werden, benötigt die grosstechnische Herstellung vergleichsweise erheblich mehr Zeit. Damit verbunden resultiert ein ungleich höherer Eintrag an Aktivierungs- und Erhaltungsenergien für den zu vermeidenden Prozess der Folgeproduktentstehung. So führt die klassische wässrige und wässrig-alkoholische Extraktion industrieller Mengen an Phyllanthus Droge zu einem dramatischen Verlust an Ellagitanninen wie auch Phyllanthinen, bedingt durch obige Einflüsse.

Ferner ist bekannt, daß im Rahmen der grosstechnischen Extraktherstellung grosse Mengen an Primärextraktionsflüssigkeit anfallen, die durch zunächst teilweises Entfernen (Destillation) des Extraktionsmittels zu einem dickflüssigen Extrakt umgewandelt werden (Softextrakt). Dieser Dickextrakt ist durch einen vergleichsweise hohen Feststoffgehalt charakterisiert (vorzugsweise 20-40% Feststoffanteil). Auch dieser Prozesschritt führt bei klassischer Ausführungsweise zu massiven Verlusten an Ellagitanninen und Phyllanthinen. Bedingt durch den Entzug des Lösungsmittels treten Sedimentationen und Flotationen auf, da das Löslichkeitsprodukt verschiedenster Inhaltsstoffe überschritten wird. Extraktivstoffe setzen sich an Maschinenteilen, die für die Wärmeübertragung verantwortlich zeichnen (Heatexchanger), ab, kleben an, brennen fest. Es bauen sich Schichten auf, die nicht von der Verdampfungskälte des evaporierenden Lösungsmittels schützbar sind. Auf diese Weise entstehen Folgeprodukte nicht bekannter Aktivität einerseits und resultieren Nativstoffverluste andererseits.

Auch die folgenden Eigenschaften werden von den im Stand der Technik bekannten Verfahren nicht erfüllt: Der Prozess muß valide sein, darf keine unkontrollierbaren kritischen Parameter aufweisen und muß im grosstechnischen Maßstab durchführbar sein. Der Wirkstoff soll trocken, in festem Aggregatzustand und pulverförmig darstellbar sein. Pharmazeutisch akzeptable Hilfsstoffe, die dieses Ziel erreichbar machen, dürfen verwendet werden. Der Wirkstoff muss bei geeigneten Lagerungsbedingungen hinsichtlich seiner chemischen, biologischen und physikalischen Eigenschaften stabil sein. Der Wirkstoff muss in eine pharmazeutisch akzeptable Arzneiformulierung integrierbar sein (vorzugsweise Filmtablette, Kapsel, Dragee).

Der Erfindung lag somit die Aufgabe zugrunde, ein Verfahren bereitzustellen, das die vorstehend erörterten Probleme löst.

Die Lösung der Aufgabe erfolgt durch die in den Ansprüchen gekennzeichneten Ausführungsformen.

Somit betrifft die vorliegende Erfindung ein Verfahren zur Herstellung eines Extraktes aus Phyllanthus, wobei man (a) Phyllanthusbestandteile mit einem Ethanol/Wassergemisch von 5-85 % m/m extrahiert, dem ein Schwermetallchelator in einer Konzentration von 0,001-3% m/m zugesetzt ist; (b) den in Schritt (a) erhaltenen Primärextrakt mit (ba) indischem Sterculiagummi in einer Endkonzentration von 0,5-5,0% mm, bezogen auf die Summe der Extraktivstoffe, oder (bb) einem oder mehreren Polymeren sowie impendierbaren und/oder löslichen Substanz(en) versetzt und konzentriert; und (c) den in Schritt (b) erhaltenen konzentrierten Extrakt trocknet.

Unter dem Bergriff "Phyllanthusbestandteile", wie erfindungsgemäß verwendet, fallen alle Bestandteile der gesamten Pflanze, wie z.B. Blätter, Rinde, Blüten, Stengel, Samen, Früchte, Äste, Stämme, Wurzeln, Holz, sowie Teile davon. Diese Phyllanthusbestandteile können dieselben, ähnliche oder nicht verwandte Inhaltsstoffe aufweisen. Es können verschiedene Phyllanthusbestandteile einzeln oder miteinander sowie verschiedene Phyllanthusbestandteile von verschiedenen Phyllanthusarten einzeln oder miteinander kombiniert in dem erfindungsgemäßen Verfahren eingesetzt werden. Unter "mehreren" Phyllanthusbestandteilen ist auch die Gesamtheit der Phyllanthúsbestandteile, beispielsweise in Form von ganzen Pflanzen zu verstehen. Die Phyllanthusbestandteile können nach Vorbehandlung oder ohne Vorbehandlung im erfindungsgemäßen Verfahren verwendet werden. Eine Vorbehandlung umfaßt beispielsweise Vorgänge wie das Trocknen, beispielsweise von Blättern.

Durch die Einführung neuer Verfahrensschritte bei der Aufbereitung von Phyllanthus sowie durch eine neuartige Kombination von Verfahrensschritten stellt die Erfindung erstmalig ein Verfahren bereit, das bei großtechnischer Durchführung reproduzierbare und valide Pflanzenextrakte bereitstellt, in denen pharmakologisch wirksame Pflanzenbestandteile in aktiver Form im wesentlichen erhalten bleiben. So wurde erfindungsgemäß überraschenderweise gefunden, daß die vorstehend diskutierte Polymerisation zu unlöslichen Makromolekülen mit dem damit verbundenen unerwünschten Verlust an pharmakologisch aktiven Ellagitanninen und Phyllanthinen durch den Zusatz von Schwermetallchelatoren zum Extraktionsmittel wirkungsvoll unterbunden wird.

Die oben geschilderte Überschreitung des Löslichkeitsproduktes sowohl polarer als auch apolarer Substanzen lässt sich in frühen Stadien der Extrakteindickung deutlich herauszögern, wenn ein Lösungsmittelgemisch mittlerer Polarität gewählt wird. Aus diesem Grunde wird in der vorliegenden Erfindung ein Ethanol\Wassergemisch, bevorzugt von 35-45%m/m eingesetzt. Diese Massnahme ist allerdings nicht ausreichend und verhindert den geschilderten Folgeproduktentstehungsprozess nur unzureichend. Überraschenderweise lässt sich durch Zusatz von Indischem Sterculiagummi die Sedimentation als auch die Flotation von Extraktivstoffen während der gesamten Konzentrierungsphase wirkungsvoll verhindern. Wie überraschenderweise entdeckt, liegt dieses Phänomen an der Eigenschaft des Polymers einerseits noch in 45%m/m Ethanol homogen zu quellen und andererseits gegenüber den Ellagitanninen innert zu sein. Durch diese Eigenschaften gelingt es, grosstechnische Mengen an Dickextrakt zu gewinnen, ohne daß die geschilderten kritischen Phasentrennungen auftreten. Pharmazeutisch akzeptable alternative Polymere wie beispielsweise Polyvinylpyrrolidone oder Hydroxypropyl-, Ethyl und Methylcellulosen lösen sich zwar in 45% m/m Ethanol, führen aber zu Ausfällungen mit Ellagitanninen, Polymere vom Typ des Gummia arabicums oder Traganths sind in 45% m/m Ethanol nicht hydratisierbar und fallen als harzige Gummen aus.

In einer bevorzugten Ausführungsform wird in Schritt (a) für die Extraktion ein Ethanol/Wassergemisch von 35-45% m/m eingesetzt.
35-45% m/m Ethanol ist bevorzugt da er bei ausreichender Lipophilie zur optimalen Extraktion den direkten Schutzeintritt des Sterculiagummis erlaubt.

In einer weiteren bevorzugten Ausführungsform wird in Schritt (a) der Schwermetallchelator in einer Konzentration von 0,1-1,0% m/m zugesetzt.

In einer zusätzlich bevorzugten Ausführungsform wird in Schritt (ba) der Primärextrakt mit indischem Sterculiagummi in einer Endkonzentration von 0,7-1,3% m/m versetzt.

Ferner ist/sind in einer weiteren bevorzugten Ausführungsform in Schritt (bb) die Substanz(en) ein pharmazeutisch verträgliches Polysaccharid/pharmazeutisch verträgliche Polysaccharide in einer Endkonzentration von 2-50% m/m, bezogen auf die Summe der Extraktivstoffe.

In einer besonders bevorzugten Ausführungsform liegt die Endkonzentration des Polysaccharids/der Polysaccharide im Bereich von 1-10% m/m.

In einer bevorzugten Ausführungsform führt man nach Schritt (a) und vor Schritt (b) (a) eine Filtration mit dem in Schritt (a) erhaltenen Primärextrakt durch, wobei der Filter ein Ausschlußvolumen von 0,05-0,5µm aufweist.

Phyllanthus amarus enthält als Naturprodukt mehr oder weniger häufig und in mehr oder weniger ausgeprägter Konzentration bakterielle Endosporen, Pilzsporen, die auch bei einer alkoholhaltigen Extraktionsweise nicht zuverlässig abgetötet werden. Da bekanntermassen eine Reihe von Inhaltstoffen von Phyllanthus amarus thermolabil sind, kommen thermische Verfahren, deren Intensität zur Abtötung dieser Sporen zuverlässig ausreichen würden, für die Entfernung dieser Kontaminanten nicht in Frage. Überraschenderweise wurde gefunden, dass sich die nach obigen ersten Verfahrensschritt erhältliche Extraktionsflüssigkeit unter Zusatz von mehrbasigen Säuren, bzw. deren Salzen, bevorzugt Dinatriumhydrogencitrat, ohne (oder nahezu ohne) Substanzverluste filtrieren, vorzugsweise ultrafiltrieren lässt, wobei die Porengrösse so gewählt ist, das ein Durchtritt der Sporen aufgrund ihrer Grösse ausgeschlossen ist. Ohne den Zusatz obigen Agens treten im Verlauf der Filtration Polymerisationsprozesse auf, die zu grobflockigen Ausfällungen auf der Filtrat- und Retentatseite führen und neben einer offensichtlichen Folgeproduktentstehung die Poren der Anlage verstopfen und den Filtrationsprozess zum Erliegen bringen.

In einer besonders bevorzugten Ausführungsform weist der Filter ein Ausschlußvolumen von 0,1-0,3 µm auf.

In einer weiteren besonders bevorzugten Ausführungsform ist die Filtration eine Ultrafiltration.

In einer zusätzlich besonders bevorzugten Ausführungsform setzt man in Schritt (a) oder vor Schritt (b) ein Lipoid in einer Endkonzentration von 1-100% m/m, bezogen auf die Extraktivstoffe, zu.

Durch den Zusatz von Lipoiden vor der Filtration werden Kontaminationen an lipophilen organischen Verunreinigungen (beispielsweise Dioxine, Aflatoxine Organochlorpestizide oder polychlorierte Biphenyle) angereichert und auf diese Weise aus der Extraktionsflüssigkeit entfernt. Durch die Tröpfchengrösse der beladenen Lipoide bedingt werden diese nämlich wie auch die Sporen im Retentat zurückgehalten und man erhält eine hochreine Wirkstofflösung auf der Filtratseite.

Ganz besonders bevorzugt ist das Lipoid ausgesucht aus der Gruppe Pflanzenöle, Wachse und Fettsäuren.

In einer bevorzugten Ausführungsform ist der Schwermetallchelator eine mehrbasige organische Säure oder deren Salz.

In einer besonders bevorzugten Ausführungsform ist die mehrbasige organische Säure Dinatriumhydrogenzitrat.

In einer bevorzugten Ausführungsform versetzt man den in Schritt (b) erhaltenen konzentrierten Extrakt vor der Trocknung mit einem oder mehreren pharmazeutisch verträglichen Hilfsstoff(en).

Pharmazeutisch verträgliche Hilfstoffe schließen pharmazeutisch verträgliche Träger und pharmazeutisch verträgliche Verdünnungsmittel ein. Bevorzugte Beispiele für derartige Hilfstoffe sind Maltodextrin und hochdisperses Siliziumdioxid.

Weitere Beispiele geeigneter Träger sind dem Fachmann bekannt und in internationalen Arzneibüchern als pharmazeutische Hilfsstoffe monographiert.

In einer weiteren bevorzugten Ausführungsform führt man die Trocknung in Schritt (c) in Gegenwart von einem oder mehreren pharmazeutisch verträglichen Hilfsstoff(en) durch.

Darüber hinaus betrifft die vorliegende Erfindung ein Verfahren zu Herstellung eines Arzneimittels, eines Nahrungsergänzungsmittels oder eines Medizinproduktes, wobei man die Schritte der erfindungsgemäßen Verfahren durchführt und wobei man den in Schritt (c) erhaltenen getrockneten Extrakt mit einem oder mehreren pharmazeutisch verträglichen Hilfsstoff(en) formuliert.

Die Arzneimittel können einem Individuum in geeigneter Dosierung verabreicht werden. Eine Verabreichung kann oral oder parenteral erfolgen, z.B. auf intravenösem, intraperitonealem, subkutanem, perinodalem, intramuskulärem, topischem, intradermalem, intranasalem, oralem oder intrabronchialem Wege oder über einen Katheter an einer Stelle in einer Arterie. Die Höhe der Dosierung wird von dem behandelnden Arzt bestimmt und hängt im wesentlichen von den klinischen Faktoren ab. Diese Faktoren sind in der Medizin und der Wissenschaft bekannt und umfassen beispielsweise die Körpergröße bzw. das Gewicht, die Körperoberfläche, das Alter, das Geschlecht, und den allgemeinen Gesundheitszustand des Patienten, die spezielle zu verabreichende Zusammensetzung, die Behandlungsdauer, die Art der Verabreichung und die eventuelle gleichzeitige Behandlung mit anderen Arzneimittel. Eine typische Dosis kann z.B. in einem Bereich zwischen 0,001 und 5000 mg Extraktivstoffen liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 100 mg- und 1000 mg-Einheiten pro Tag befinden. Wird die Zusammensetzung intravenös verabreicht, was nicht bevorzugt empfohlen wird, um die Gefahr einer anaphylaktischen Reaktionen zu minimieren, sollte sich die Dosis in einem Bereich zwischen 1 µgund 10 mg-Einheiten pro Kilogramm Körpergewicht pro Minute befinden.

Die vorliegende Erfindung betrifft weiterhin ein Verfahren zu Herstellung eines Arzneimittels, eines Nahrungsergänzungsmittels oder eines Medizinproduktes, wobei man die Schritte der erfindungsgemäßen Verfahren durchführt und wobei man die Trocknung in Schritt (c) in Gegenwart von einem oder mehreren pharmazeutisch verträglichen Hilfstoff(en) durchführt.

Des weiteren betrifft die vorliegende Erfindung ein Verfahren zu Herstellung eines Arzneimittels, eines Nahrungsergänzungsmittels oder eines Medizinproduktes, wobei man die Schritte der erfindungsgemäßen Verfahren durchführt und wobei man den oder die pharmazeutisch verträglichen Hilfstoff(en) vor der Trocknung in Schritt (c) zusetzt.

In einer bevorzugten Ausführungsform sind die Hilfsstoffe Maltodextrin und/oder hochdisperses Siliziumdioxid.

In einer weiteren bevorzugten Ausführungsform erfolgt die Trocknung mittels Zerstäubungs-, Band- oder Gefriertrocknung.

Zusätzlich ist in einer bevorzugten Ausführungsform der Phyllanthus Phyllanthus amarus.

In einer besonders bevorzugten Ausführungsform ist der Phyllanthus amarus Phyllanthus amarus Schumach et Thonn.

Des weiteren betrifft die vorliegende Erfindung ein Phyllanthusextrakt erhältlich nach dem erfindungsgemäßen Verfahren.

Darüber hinaus betrifft die vorliegende Erfindung auch ein Arzneimittel erhältlich nach dem erfindungsgemäßen Verfahren.

Zusätzlich betrifft die vorliegende Erfindung ein Arzneimittel enthaltend einen Phyllantusextrakt hergestellt nach dem erfindungsgemäßen Verfahren.

In einer bevorzugten Ausführungsform ist die Darreichungsform eine Tablette, ein Dragee, eine Hartgelatinekapsel oder eine Weichgelatinekapsel.

In einer besonders bevorzugten Ausführungsform ist die Tablette eine Filmtablette.

Die Beispiele erläutern die Erfindung.

### Beispiel 1: Extraktion von Phyllanthus amarus-Blättern

Getrocknete Phyllanthus amarus-Blätter wurden in eine Extraktionsanlage (Stahlgefäß) eingefüllt. Als Extraktionsmittel wurde 50% v/v EtOH eingesetzt. Dem Ansatz wurde ferner Dinatriumhydrogenzitrat in einer Endkonzentration von 0,1-1,0% m/m zugesetzt. Der EtOH-Gehalt wurde mittels Dichtemessung überprüft und entsprach 35-45% m/m. Das Verhältnis von Droge und Lösungsmittel betrug 1:10 (+/- 3 Lösungsmittel). Die Blätter wurden 1 Stunde bei einer Temperatur zwischen 30 und 50°C extrahiert. Anschließend wurde die Miscela über einen Filter mit Wasser (entspricht drei Teilen Drogen) nachgewaschen und abgepreßt. Danach wurde das Gemisch durch eine Membran mit einem Ausschlußvolumen zwischen 0,1-0,3 µm filtriert. Zu der. Lösung wurde indisches Sterculiagummi, das zuvor in Ethanol-/Wasser oder in absolutem Ethanol gelöst wurde, zugegeben. Das Gemisch wurde aufkonzentriert. Dies geschah durch Evaporierung unter vermindertem Druck (etwa 300 mB absteigend auf 20 mB), wobei eine Temperatur von 30-60°C (+/- 5°C) eingesetzt wurde, bis das Material einen Trockengehalt von 20-40% (m/m) aufwies. Nachfolgend wurde der Weichextrakt mit Maltodextrin gemischt, bis eine homogene Suspension erhalten wurde. Anschließend wurde das Gemisch einer Sprühtrocknung unterworfen.
Die Werte der hier untersuchten Pestizide sind in Tabelle 1, die Ellagitanninwerte in Tabelle 2 erwähnt.

### Beispiel 2: Extraktion von Phyllanthus amarus-Blättern

Das Verfahren wurde gemäß Beispiel 1 mit folgenden Änderungen durchgeführt: Nach der Zugabe von Maltodextrin wurde das Gemisch in einen Kurzzeiterhitzer bei einer Temperatur von 100°C für eine Dauer von 36 Sekunden gegeben. Damit wird die mikrobielle Belastung der Droge vermindert. Das Gemisch wurde getrocknet, bis der Wassergehalt unter 5% lag. Während des Trockenvorgangs überschritt die Auslaßtemperatur der Heizeinheit 90°C (+/- 5%) nicht. Während des Trocknungsprozesses und nach dem Trocknungsprozess wurde Silica zugegeben. Das getrocknete Produkt wurde gemischt und nachfolgend gesiebt.
Die Werte der hier untersuchten Pestizide sind in Tabelle 1, die Ellagitanninwerte in Tabelle 2 erwähnt.
Aus Tabelle 2 ist ersichtlich, daß sich die Ellagitanninwerte nicht wesentlich ändern durch die Kurzzeiterhitzung.

### Beispiel 3: Extraktion von Phyllanthus amarus-Blättern

Das Verfahren wurde gemäß Beispiel 1 mit folgenden Änderungen durchgeführt: Vor der Filtration wurde dem Gemisch ein Lipid (Miglyol) beigegeben (Endkonzentration von 54,9% m/m, bezogen auf die Extraktivstoffe). Dann wurde das Gemisch durch eine Membran mit einem Ausschlußvolumen zwischen 0,1-0,3 µm filtriert. Durch die vorangehende Lipoidzugabe werden bei der Ultrafiltration Kontaminationen an lipophilen organischen Verunreinigungen entfernt. Anschließend durchgeführte Konzentration, Ausmischung und Sprühtrocknung sind in Beispiel 1 beschrieben.
Die Werte der hier untersuchten Pestizide sind in Tabelle 1, die Ellagitanninwerte in Tabelle 2 erwähnt.
Aus Tabelle 1 ist, im Vergleich zu Beispiel 1 und Beispiel 2, eine deutliche Entfernung der unerwünschten lipophilen Verunreinigungen und/oder Rückstände bis unterhalb der Detektionsgrenze erkennbar. Tabelle 2 zeigt, daß keine Entfernung der Ellagitannine stattfindet.

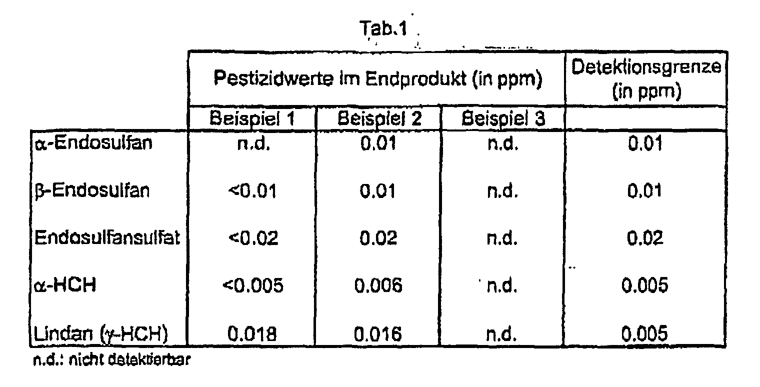

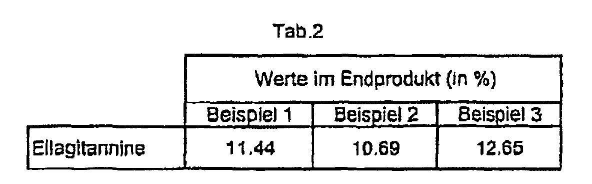

## Patentansprüche

1. Verfahren zur Herstellung eines Extraktes aus Phyllanthus, wobei man
(a) Phyllanthusbestandteile mit einem Ethanol/Wassergemisch von 5-85 % m/m extrahiert, dem ein Schwemmetallchelator in einer Konzentration von 0,001-3% m/m zugesetzt ist;
(b) den in Schritt (a) erhaltenen Primärextrakt mit
(ba) indischem Sterculiagummi in einer Endkonzentration von 0,5-5,0% m/m, bezogen auf die Summe der Extraktivstoffe, oder
(bb) einem oder mehreren Polymeren sowie impendierbaren und/oder löslichen Substanz(en)
versetzt und konzentriert; und
(c) den in Schritt (b) erhaltenen konzentrierten Extrakt trocknet.

2. Verfahren nach Anspruch 1, wobei in Schritt (a) für die Extraktion ein Ethanol/Wassergemisch von 35-45% m/m eingesetzt wird.

3. Verfahren nach Anspruch 1 oder 2, wobei in Schritt (a) der Schwermetallchelator in einer Konzentration von 0,1-1,0% m/m zugesetzt ist.

4. Verfahren nach einem der Ansprüche 1 bis 3, wobei in Schritt (ba) der Primärextrakt mit indischem Sterculiagummi in einer Endkonzentration von 0,7-1,3% m/m versetzt wird.

5. Verfahren nach einem der Ansprüche 1 bis 3, wobei in Schritt (bb) die Substanz(en) ein pharmazeutisch verträgliches Polysaccharid ist/pharmazeutisch verträgliche Polysaccharide in einer Endkonzentration von 2-50% m/m, bezogen auf die Summe der Extraktivstoffe, sind.

6. Verfahren nach Anspruch 5, wobei die Endkonzentration des Polysaccharids/der Polysaccharide im Bereich von 1-10% m/m liegt.

7. Verfahren nach einem der Ansprüche 1 bis 6, wobei man nach Schritt (a) und vor Schritt (b)
(a) eine Filtration mit dem in Schritt (a) erhaltenen Primärextrakt durchführt, wobei der Filter ein Ausschlußvolumen von 0,05-0,5 µm aufweist.

8. Verfahren nach Anspruch 7, wobei der Filter ein Ausschlußvolumen von 0,1-0,3 µm aufweist.

9. Verfahren nach Anspruch 7 oder 8, wobei die Filtration eine Ultrafiltration ist.

10. Verfahren nach einem der Ansprüche 7 bis 9, wobei man in Schritt (a) oder vor Schritt (b) ein Lipoid in einer Endkonzentration von 1-100% m/m, bezogen auf die Extraktivstoffe, zusetzt.

11. Verfahren nach Anspruch 10, wobei das Lipoid ausgesucht ist aus der Gruppe Pflanzenöle, Wachse und Fettsäuren.

12. Verfahren nach einem der Ansprüche 1 bis 11, wobei der Schwermetallchelator eine mehrbasige organische Säure oder deren Salz ist.

13. Verfahren nach Anspruch 12, wobei die mehrbasige organische Säure Dinatriumhydrogenzitrat ist.

14. Verfahren nach einem der Ansprüche 1 bis 13, wobei man den in Schritt (b) erhaltenen konzentrierten Extrakt vor der Trocknung mit einem oder mehreren pharmazeutisch verträglichen Hilfsstoff(en) versetzt.

15. Verfahren nach einem der Ansprüche 1 bis 13, wobei man die Trocknung in Schritt (c) in Gegenwart von einem oder mehreren pharmazeutisch verträglichen Hilfsstoff(en) durchführt.

16. Verfahren zur Herstellung eines Arzneimittels, eines Nahrungsergänzungsmittels oder eines Medizinproduktes, wobei man die Schritte der Verfahren nach einem der Ansprüche 1 bis 13 durchführt und wobei man den in Schritt (c) erhaltenen getrockneten Extrakt mit einem oder mehreren pharmazeutisch verträglichen Hilfsstoff(en) formuliert.

17. Verfahren zur Herstellung eines Arzneimittels, eines Nahrungsergänzungsmittels oder eines Medizinproduktes, wobei man die Schritte der Verfahren nach einem der Ansprüche 1 bis 13 durchführt und wobei man die Trocknung in Schritt (c) in Gegenwart von einem oder mehreren pharmazeutisch verträglichen Hilfstoff(en) durchführt.

18. Verfahren zur Herstellung eines Arzneimittels, eines Nahrungsergänzungsmittels oder eines Medizinproduktes, wobei man die Schritte der Verfahren nach einem der Ansprüche 1 bis 13 durchführt und wobei man den oder die pharmazeutisch verträglichen Hilfstoff(e) vor der Trocknung in Schritt (c) zusetzt.

19. Verfahren nach einem der Ansprüche 14 bis 18, wobei die Hilfsstoffe Maltodextrin und/oder hochdisperses Siliziumdioxid sind.

20. Verfahren nach einem der Ansprüche 1 bis 19, wobei die Trocknung mittels Zerstäubungs-, Band- oder Gefriertrocknung erfolgt.

21. Verfahren nach einem der Ansprüche 1 bis 20, wobei der Phyllanthus Phyllanthus amarus ist.

22. Verfahren nach Anspruch 21, wobei der Phyllanthus amarus Phyllanthus amarus Schumach et Thonn ist.

23. Phyllanthusextrakt erhältlich nach dem Verfahren nach einem der Ansprüche 1 bis 15, 21 und 22.

24. Arzneimittel erhältlich nach dem Verfahren nach einem der Ansprüche 14 bis 22.

25. Arzneimittel enthaltend einen Phyllantusextrakt hergestellt nach dem Verfahren nach einem der Ansprüche 1 bis 15, 21 und 22.

26. Arzneimittel nach Anspruch 24 oder 25, wobei die Darreichungsform eine Tablette, ein Dragee, eine Hartgelatinekapsel oder eine Weichgelatinekapsel ist.

27. Arzneimittel nach Anspruch 26, wobei die Tablette eine Filmtablette ist.

## Claims

1. A method for the production of an extract of Phyllanthus wherein
(a) Phyllanthus components are extracted with an ethanol/water mixture of 5-85% m/m to which a heavy-metal chelator is added at a concentration of 0.001-3% m/m;
(b) the primary extract obtained in step (a) is added to and concentrated with
(ba) Indian Sterculia gum at a final concentration of 0.5-5.0% m/m relative to the sum of the extractive substances or
(bb) one or more polymers and impendable and/or soluble substance(s); and
(c) the concentrated extract obtained in step (b) is dried.

2. The method according to claim 1, wherein in step (a) an ethanol/water mixture of 35-45% m/m is added for the extraction.

3. The method according to claim 1 or 2, wherein in step (a) the heavy-metal chelator is added at a concentration of 0.1-1.0% m/m.

4. The method according to any one of claims 1 to 3, wherein in step (ba) Indian Sterculia gum at a final concentration of 0.7-1.3% m/m is added to the primary extract.

5. The method according to any one of claims 1 to 3, wherein in step (bb) the substance(s) is/are a pharmaceutically acceptable polysaccharide/pharmaceutically acceptable polysaccharides at a final concentration of 2-50% m/m relative to the sum of the extractive agents.

6. The method according to claim 5, wherein the final concentration of the polysaccharide(s) is in the range of 1-10% m/m.

7. The method according to any one of claims 1 to 6, wherein after step (a) and before step (b) (a) a filtration is carried out with the primary extract obtained in step (a), wherein the filter has an exclusion volume of 0.05-0.5 µm.

8. The method according to claim 7, wherein the filter has an exclusion volume of 0.1-0.3 µm.

9. The method according to claim 7 or 8, wherein the filtration is an ultrafiltration.

10. The method according to any one of claims 7 to 9, wherein in step (a) or before step (b) a lipoid is added at a final concentration of 1-100% m/m relative to the extractive agents.

11. The method according to claim 10, wherein the lipoid is selected from the group of plant oils, waxes and fatty acids.

12. The method according to any one of claims 1 to 11, wherein the heavy-metal chelator is a polyprotic organic acid or the salt thereof.

13. The method according to claim 12, wherein the multi-base organic acid is disodiumhydrogen citrate.

14. The method according to any one of claims 1 to 13, wherein one or more pharmaceutically acceptable adjuvant(s) are added to the concentrated extract obtained in step (b) before the drying.

15. The method according to any one of claims 1 to 13, wherein the drying in step (c) is carried out in the presence of one or more pharmaceutically acceptable adjuvant(s).

16. A method for preparing a pharmaceutical composition, a food supplement or a medicinal product, wherein the steps of the methods are carried out according to any one of claims 1 to 13 and wherein the dried extract obtained in step (c) is formulated with one or more pharmaceutically acceptable adjuvant(s).

17. A method for the production of a pharmaceutical preparation, a food supplement or a medicinal product, wherein the steps of the methods are carried out according to any one of claims 1 to 13 and wherein the drying in step (c) is carried out in the presence of one or more pharmaceutically acceptable adjuvant(s).

18. A method for the production of a pharmaceutical preparation, a food supplement or a medicinal product, wherein the steps of the methods are carried out according to any one of claims 1 to 13 and wherein the pharmaceutically acceptable adjuvant(s) are added before the drying in step (c).

19. The method according to any one of claims 14 to 18, wherein the adjuvants are maltodextrin and/or highly dispersed silicon dioxide.

20. The method according to any one of claims 1 to 19, wherein the drying is carried out by means of spray, band or freeze drying.

21. The method according to any one of claims 1 to 20, wherein the Phyllanthus is Phyllanthus amarus.

22. The method according to claim 21, wherein the Phyllanthus amarus is the Phyllanthus amarus Schumach et Thonn.

23. A Phyllanthus extract obtainable according to the method according to any one of claims 1 to 15, 21 and 22.

24. Pharmaceutical preparation obtainable according to the method of any one of claims 14 to 22.

25. Pharmaceutical preparation containing a Phyllanthus extract produced according to the method of any one of claims 1 to 15, 21 and 22.

26. Pharmaceutical preparation according to claim 24 or 25, wherein the dosage form is a tablet, a sugar-coated tablet, a hard gelatine capsule or a soft gelatine capsule.

27. Pharmaceutical preparation according to claim 26, wherein the tablet is a coated tablet.

## Revendications

1. Procédé pour la fabrication d'un extrait de phyllanthus, dans lequel
(a) on extrait des composants de phyllanthus avec un mélange éthanol/eau de 5-85 % m/m, auquel on a ajouté un chélateur de métaux lourds en une concentration de 0,001-3 % m/m ;
(b) on additionne et on concentre l'extrait primaire obtenu à l'étape (a) avec
(ba) de la gomme de sterculia indienne en une concentration finale de 0,5-5,0 % m/m, rapportée à la somme des matières extractives, ou
(bb) un ou plusieurs polymère(s) ainsi qu'une ou plusieurs substance(s) pouvant être mise(s) én suspension et/ou en solution, et
(c) on sèche l'extrait concentré obtenu à l'étape (b).

2. Procédé selon la revendication 1, dans lequel on utilise pour l'extraction dans l'étape (a) un mélange éthanol/eau de 35-45 % m/m.

3. Procédé selon la revendication 1 ou 2, dans lequel on ajoute le chélateur de métaux lourds dans l'étape (a) en une concentration de 0,1-1,0 % m/m.

4. Procédé selon l'une quelconque des revendications 1 à 3, dans lequel on additionne l'extrait primaire dans l'étape (ba) avec la gomme de sterculia indienne en une concentration finale de 0,7-1,3 % m/m.

5. Procédé selon l'une quelconque des revendications 1 à 3, dans lequel, dans l'étape (bb), la substance est un polysaccharide compatible au point de vue pharmaceutique / les substances sont des polysaccharides compatibles au point de vue pharmaceutique, en une concentration finale de 2-50 % m/m, rapportée à la somme des matières extractives.

6. Procédé selon la revendication 5, dans lequel la concentration finale du polysaccharide / des polysaccharides se situe dans la plage de 1-10 % m/m.

7. Procédé selon l'une quelconque des revendications 1 à 6, dans lequel on effectue après l'étape (a) et avant l'étape (b)
(a) une filtration avec l'extrait primaire obtenu à l'étape (a), dans laquelle le filtre présente un volume d'exclusion de 0,05-0,5 µm.

8. Procédé selon la revendication 7, dans lequel le filtre présente un volume d'exclusion de 0,1-0,3 µm.

9. Procédé selon la revendication 7 ou 8, dans lequel la filtration est une ultrafiltration.

10. Procédé selon l'une quelconque des revendications 7 à 9, dans lequel on ajoute, à l'étape (a) ou avant l'étape (b), un lipoïde en une concentration finale de 1-100 % m/m, rapportée aux matières extractives.

11. Procédé selon la revendication 10, dans lequel le lipoïde est sélectionné parmi le groupe composé des huiles végétales, des cires et des acides gras.

12. Procédé selon l'une quelconque des revendications 1 à 11, dans lequel le chélateur de métaux lourds est un acide organique polybasique ou un sel de celui-ci.

13. Procédé selon la revendication 12, dans lequel l'acide organique polybasique est l'hydrogénocitrate disodique.

14. Procédé selon l'une quelconque des revendications 1 à 13, dans lequel on additionne l'extrait concentré obtenu à l'étape (b) , avant le séchage, avec une ou plusieurs substance(s) secondaire(s) compatible(s) au point de vue pharmaceutique.

15. Procédé selon l'une quelconque des revendications 1 à 13, dans lequel on effectue le séchage dans l'étape (c) en présence d'une ou de plusieurs substance(s) secondaire(s) compatible(s) au point de vue pharmaceutique.

16. Procédé pour la fabrication d'un médicament, d'un complément alimentaire ou d'un produit médicinal, dans lequel on exécute les étapes de procédé selon l'une quelconque des revendications 1 à 13 et dans lequel on formule l'extrait séché obtenu à l'étape (c) avec une ou plusieurs substance(s) secondaire(s) compatible(s) au point de vue pharmaceutique.

17. Procédé pour la fabrication d'un médicament, d'un complément alimentaire ou d'un produit médicinal, dans lequel on exécute les étapes de procédé selon l'une quelconque des revendications 1 à 13 et dans lequel on effectue le séchage à l'étape (c) en présence d'une ou de plusieurs substance(s) secondaire(s) compatible(s) au point de vue pharmaceutique.

18. Procédé pour la fabrication d'un médicament, d'un complément alimentaire ou d'un produit médicinal, dans lequel on exécute les étapes de procédé selon l'une quelconque des revendications 1 à 13 et dans lequel on ajoute la ou les substance(s) secondaire(s) compatible(s) au point de vue pharmaceutique avant le séchage à l'étape (c).

19. Procédé selon l'une quelconque des revendications 14 à 18, dans lequel les substances secondaires sont la maltodextrine et/ou du dioxyde de silicium hautement dispersible.

20. Procédé selon l'une quelconque des revendications 1 à 19, dans lequel le séchage est effectué par séchage par pulvérisation, par séchage à bande ou par séchage par lyophilisation.

21. Procédé selon l'une quelconque des revendications 1 à 20, dans lequel le phyllanthus est le phyllanthus amarus.

22. Procédé selon la revendication 21, dans lequel le phyllanthus amarus est le phyllanthus amarus Schumach et Thonn.

23. Extrait de phyllanthus pouvant être obtenu par le procédé selon l'une quelconque des revendications 1 à 15, 21 et 22.

24. Médicament pouvant être obtenu par le procédé selon l'une quelconque des revendications 14 à 22.

25. Médicament contenant un extrait de phyllanthus fabriqué par le procédé selon l'une quelconque des revendications 1 à 15, 21 et 22.

26. Médicament selon la revendication 24 ou 25, dans lequel la forme d'administration est un comprimé, une dragée, une gélule dure ou une gélule souple.

27. Médicament selon la revendication 26, dans lequel le comprimé est un comprimé pelliculé.
